# FASCICULE DE BREVET EUROPEEN

(11) **EP 3 212 771 B1**
(45) Date de publication et mention de la délivrance du brevet: **05.12.2018**
(21) Numéro de dépôt: 15798208.3
(22) Date de dépôt: 29.10.2015
(51) Int. Cl.: G01N 33/50, C12N 5/071

(54) **SPHEROIDES MIXTES DE MELANOCYTES ET DE KERATINOCYTES**
GEMISCHTE SPHÄROIDE VON MELANOZYTEN UND KERATINOZYTEN
MIXED SPHEROIDS OF MELANOCYTES AND KERATINOCYTES

(30) Priorité: 30.10.2014 FR 1460467
(43) Date de publication de la demande: 06.09.2017
(73) Titulaire: Syntivia, 31106 Toulouse Cedex 1 (FR); Centre Hospitalier Universitaire De Toulouse, 31059 Toulouse Cedex 9 (FR); Université Paul Sabatier Toulouse III, 31062 Toulouse Cedex 9 (FR); Centre National de la Recherche Scientifique, 75016 Paris (FR)
(72) Inventeur: NORLUND, Marine, F-31830 Plaisance du Touch (FR); DUCOMMUN, Bernard, F-31450 Belberaud (FR); BEDOS, Philippe, F-31450 Donneville (FR); LOBJOIS, Valérie, F-32600 Pujaudran (FR)
(74) Mandataire: Gevers & Orès
(86) Numéro de dépôt international: PCT/IB2015/058350
(87) Numéro de publication internationale: WO 2016/067240

(56) Documents cités:
- EP-A1- 1 878 790
- WO-A1-01/79453
- LEI T C ET AL: "A Melanocyte-Keratinocyte Coculture Model to Assess Regulators of Pigmentation in Vitro", ANALYTICAL BIOCHEMISTRY, ACADEMIC PRESS INC, NEW YORK, vol. 305, no. 2, 15 juin 2002 (2002-06-15) , pages 260-268, XP027227027, ISSN: 0003-2697 [extrait le 2002-06-15]
- LING LI ET AL: "The Three-Dimensional Human Skin Reconstruct Model: a Tool to Study Normal Skin and Melanoma Progression", JOURNAL OF VISUALIZED EXPERIMENTS, no. 54, 3 août 2011 (2011-08-03), XP055184451, DOI: 10.3791/2937
- LIMAT A ET AL: "OUTER ROOT SHEATH (ORS) CELLS ORGANIZE INTO EPIDERMOID CYST-LIKE SPHEROIDS WHEN CULTURED INSIDE MATRIGEL: A LIGHT-MICROSCOPIC AND IMMUNOHISTOLOGICAL COMPARISON BETWEEN HUMAN ORS CELLS AND INTERFOLLICULAR KERATINOCYTES", CELL AND TISSUE RESEARCH, BERLIN, DE, vol. 275, no. 1, 1 janvier 1994 (1994-01-01), pages 169-176, XP009037056, DOI: 10.1007/BF00305384
- LIN ET AL: "Enhanced cell survival of melanocyte spheroids in serum starvation condition", BIOMATERIALS, ELSEVIER SCIENCE PUBLISHERS BV., BARKING, GB, vol. 27, no. 8, 1 mars 2006 (2006-03-01), pages 1462-1469, XP005193213, ISSN: 0142-9612, DOI: 10.1016/J.BIOMATERIALS.2005.08.031

## Description

La présente invention se rapporte à la mise au point d'un modèle d'évaluation d'actifs ciblant l'épiderme. Elle vise plus spécifiquement la préparation de sphéroïdes mixtes de mélanocytes et de kératinocytes (aussi désigné par sphéroïdes mixtes dans ce qui suit) reproduisant les interactions cellulaires existantes au sein de l'épiderme, les sphéroïdes en tant que tels et leurs utilisations.

L'épiderme est la couche la plus superficielle de la peau. Il est majoritairement constitué de kératinocytes qui prolifèrent et se différencient jusqu'à donner des cellules mortes appelées cornéocytes qui s'éliminent par desquamation. Les mélanocytes, cellules spécialisées responsables de la production du pigment mélanique, se situent dans l'épiderme au niveau de la lame basale. La mélanine produite par les mélanocytes s'accumule dans des vésicules appelées mélanosomes qui sont transférées aux kératinocytes par leurs nombreuses extensions dendritiques. La pigmentation de la peau est la résultante de ce processus, appelé mélanogénèse, conduisant à la production de mélanine.

L'épiderme remplit plusieurs fonctions incluant notamment la pigmentation de la peau et la fonction barrière, principalement assurée par la couche cornée. Ces fonctions peuvent être modulées par des actifs.

A titre d'exemple, des produits dépigmentants sont disponibles dans le commerce à des fins cosmétiques ou dermatologiques pour le traitement de troubles hyper-pigmentaires de type melasma ou lentigo solaire. Le plus connu d'entre eux, l'hydroquinone, présente de nombreux effets secondaires et est aujourd'hui interdit en cosmétique. La mise en évidence de nouveaux composés pouvant représenter des alternatives à l'hydroquinone nécessite l'utilisation de modèles biologiques permettant d'évaluer leur activité dépigmentante.

A l'heure actuelle, les modèles de co-culture de kératinocytes et de mélanocytes sont les plus utilisés dans ce domaine. Ils permettent de suivre différents aspects de la biologie de l'épiderme mais ne rendent pas compte du microenvironnement tridimensionnel de l'épiderme. Il existe également des modèles de peaux reconstruites mimant de façon plus fidèle le comportement de ces deux types cellulaires au sein de l'épiderme. Cependant les peaux reconstruites sont des modèles très lourds à mettre en oeuvre et également trop onéreux pour être utilisés en routine pour du criblage de molécules.

Il apparait donc nécessaire de mettre au point des modèles tridimensionnels d'épiderme rapides à préparer et faciles à manipuler pour l'évaluation de nouveaux actifs.

C'est dans ce contexte que la Demanderesse a mis au point des sphéroïdes comportant une population cellulaire mixte de kératinocytes et de mélanocytes.

La présente invention se rapporte en premier lieu à un procédé de préparation de sphéroïdes mixtes de kératinocytes et de mélanocytes caractérisé en ce qu'il comprend :
(a) la mise en suspension de kératinocytes avec des mélanocytes dans un milieu de culture supplémenté d'une préparation de protéines de la matrice extra-cellulaire, de préférence, cette préparation est présente à une concentration comprise entre 1 à 95% en volume/volume (pourcentage exprimé en volume de préparation par rapport au volume total du milieu de culture), de préférence entre 10 et 75% en volume/volume, et d'une quantité inférieure ou égale à 10 mM, de préférence comprise entre 0,1 et 1 mM, d'un sel choisi parmi le calcium, le manganèse ou le magnésium, de préférence, il s'agit du calcium ;
(b) la formation des sphéroïdes à partir du mélange obtenu à l'étape (a) ; et
(c) l'incubation des sphéroïdes obtenus à l'étape (b).

Les cellules, kératinocytes et mélanocytes, utilisables dans le procédé selon l'invention peuvent être saines ou malades selon l'utilisation souhaitée des sphéroïdes ; lorsqu'il s'agit de cellules saines, elles peuvent être des kératinocytes primaires sains du type NHEK (Normal Human Epidermal Keratinocyte) et des mélanocytes primaires sains du type NHEM (Normal Human Epidermal Melanocyte) provenant de biopsie ou de source commerciale. Le procédé peut être conduit avec des mélanocytes de tout phototype compris entre 1 et 6 ; là encore, l'homme du métier choisira le phototype le plus approprié aux expérimentations envisagées.

Le ratio de kératinocytes par rapport aux mélanocytes n'est pas critique pour la préparation des sphéroïdes selon l'invention ; on préférera toutefois utiliser un ratio qui se rapproche autant que possible de celui de l'épiderme, c'est-à-dire compris entre 1 mélanocyte pour 1 kératinocyte et 1 mélanocyte pour 40 kératinocytes ; de préférence, ce ratio est compris entre 1 mélanocyte pour 2 kératinocytes et 1 mélanocyte pour 5 kératinocytes.

La quantité totale de cellules ensemencées pour la préparation d'un sphéroïde n'est pas essentielle et est adaptée en fonction de l'origine des cellules ensemencées ; à titre d'exemple, afin d'obtenir des sphéroïdes d'une dimension optimisée à partir de cellules primaires saines, il est préférable d'ensemencer entre 100 et 5000 cellules, de préférence entre 2000 et 3000 cellules, pour un sphéroïde. Les cellules sont de préférence ensemencées dans des plaques 96 puits.

La préparation de protéines de la matrice extracellulaire est de préférence une préparation d'extrait protéique issue d'un sarcome comme par exemple celle vendue par la société Corning Life Sciences sous le nom Matrigel®.

L'étape (b) est de préférence réalisée par centrifugation, par exemple, entre 50 et 2200g pendant entre 30 secondes et 15 minutes, de préférence 6 minutes.

L'étape (c) d'incubation est conduite dans des conditions classiques ; c'est-à-dire à une température comprise entre 35 et 38°C. Selon un mode de réalisation, cette étape comporte une incubation d'une durée de 1h environ dans le milieu de culture contenant la préparation de protéines de la matrice extracellulaire telle que le Matrigel®, puis le sel est ajouté et l'incubation est poursuivie de préférence pendant au moins 10h.

A titre indicatif, l'incubation de l'étape (c) peut durer entre 1 et 10 jours, de préférence entre 2 et 5 jours, selon le degré de différenciation des kératinocytes souhaité.

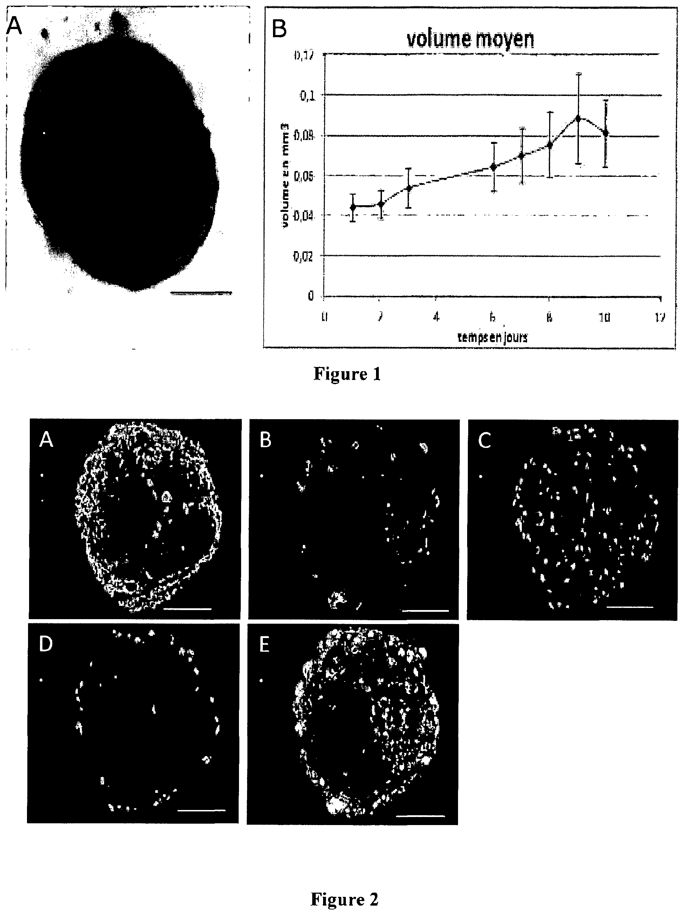

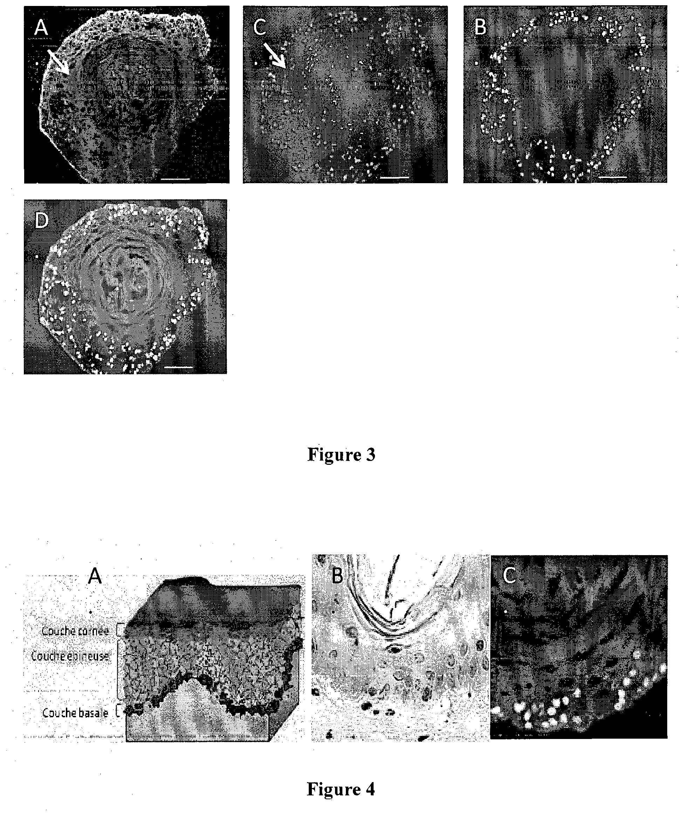

Selon une variante de l'invention, l'incubation des sphéroïdes est prolongée au-delà de 3 jours pour voir apparaitre une modification des kératinocytes (allongement des cellules, aplatissement des noyaux, accumulation de kératine) au centre des sphéroïdes (**Figures 2 à 4**). Cette caractéristique rend le modèle très intéressant puisqu'il reproduit le gradient de différenciation des kératinocytes. Le sphéroïde mixte mime ainsi une peau « à l'envers » (les cellules les plus différenciées étant au coeur du sphéroïde) avec une structure similaire aux différentes strates de la peau (**Figure 4**).

En vue de leur utilisation, il est possible de laver, par exemple à l'aide de PBS, les sphéroïdes mixtes afin d'éliminer la préparation de protéines de la matrice extracellulaire ; ce lavage est effectué de préférence après 24h à 168h d'incubation, le temps après lequel il est réalisé dépend de la maturité souhaitée des sphéroïdes mixtes.

Il est également envisageable d'éliminer le sel présent dans le milieu de culture des sphéroïdes mixtes avant leur utilisation.

Ainsi selon un mode de réalisation particulier du procédé selon l'invention, celui-ci comprend une étape (d) additionnelle de lavage des sphéroïdes mixtes, de préférence avec du PBS.

Un avantage du procédé selon l'invention est qu'il permet de produire rapidement un grand nombre de sphéroïdes ayant des caractéristiques de taille et de population cellulaire standardisées, ce qui permet la mise en oeuvre d'essais de criblage en série et de comparer des résultats obtenus.

Diverses tentatives de préparation de sphéroïdes présentées dans la partie expérimentale ont permis de mettre en évidence les caractéristiques essentielles du procédé. Ainsi, l'absence de sel et/ou de préparation de protéines de la matrice extracellulaire, contenant également en particulier des facteurs de croissance, ou encore la préparation de sphéroïde simple d'un seul des deux types cellulaires auquel serait ajouté une suspension de l'autre type cellulaire ne conduisent pas à la formation de sphéroïde.

La présente invention se rapporte également à des sphéroïdes mixtes de kératinocytes et de mélanocytes obtenus par le procédé de préparation selon l'invention ; ainsi les sphéroïdes mixtes de kératinocytes et de mélanocytes selon l'invention peuvent comprendre une préparation de protéines de la matrice extracellulaire à une concentration comprise entre 1 à 95% en volume/volume (pourcentage exprimé en volume de préparation par rapport au volume total du milieu de culture), de préférence entre 10 et 75% en volume/volume et une quantité inférieure ou égale à 10 mM, de préférence comprise entre 0,1 et 1 mM, d'un sel choisi parmi le calcium, le magnésium et le manganèse pouvant être préparés selon le procédé qui précède ; avantageusement, il s'agit de sphéroïdes ayant un diamètre apparent compris entre 100 µm et 1000 µm et une forme régulière. De préférence, les sphéroïdes comprennent un nombre de cellules compris entre 100 cellules et 5000 cellules, de préférence entre 2000 et 3000 cellules.

La présente invention se rapporte encore à des sphéroïdes mixtes qui comportent une masse centrale correspondant à une différenciation centrale des kératinocytes.

Les sphéroïdes mixtes selon l'invention reproduisent les interactions kératinocytes/kératinocytes, kératinocytes/mélanocytes avec la présence d'extensions dendritiques et la production de mélanine par les mélanocytes. Ils peuvent donc servir de modèles pour l'évaluation de l'activité de molécules actives notamment dermatologiques ou cosmétiques.

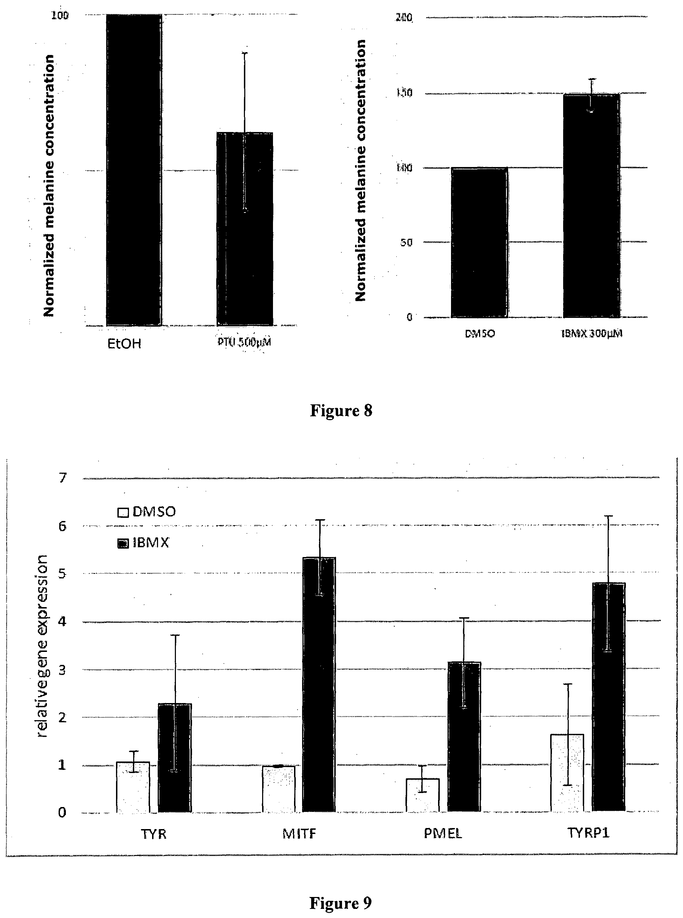

Divers tests ont été conduits avec des sphéroïdes mixtes selon l'invention : après traitement des sphéroïdes mixtes avec des composés connus pour être inducteurs (IBMX, 3-isobutyl-1-methylxanthine) et inhibiteurs (PTU, propylthiouracile) de la production de mélanine, la quantité de mélanine (dosée par spectrophotométrie) rapportée aux protéines totales a été évaluée (voir l'exemple 5 et la **Figure 8**). Une induction de 50% a été visualisée de façon reproductible après traitement à l'IBMX validant ainsi l'utilisation de l'IBMX comme molécule de référence sur le modèle sphéroïde. Le traitement au PTU, quant à lui, inhibe la production de mélanine.

Cette quantification directe a été complétée par une étude en PCR quantitative ; cette méthode permet d'analyser un grand nombre de gènes et donc de mettre en évidence les mécanismes d'action des molécules testées sur les sphéroïdes mixtes. Après 24h de traitement à l'IBMX, tous les gènes de la pigmentation étudiés (TYR, MITF, PMEL et TYRP1) ont été induits au niveau génique (voir l'exemple 6 et la **Figure 9**). Le facteur MITF intervenant dans la régulation des gènes de la pigmentation et directement impliqué dans la réponse à l'IBMX d'un point de vue mécanistique est augmenté d'un facteur 5. Les gènes de synthèse de la mélanine TYR et TYRP1 sont également induits.

Ces résultats confirment qu'il est possible de quantifier la mélanine produite dans des sphéroïdes mixtes selon l'invention suite à des traitements de référence. La quantité de mélanine est directement quantifiable par dosage de la mélanine et une modulation est obtenue suite à des traitements de références pro ou dépigmentants. De plus, une analyse transcriptionnelle est également possible, complétant ainsi l'évaluation d'actifs.

Les sphéroïdes mixtes selon l'invention présentent donc un intérêt marqué comme modèle pour évaluer des molécules pro- ou dé-pigmentantes.

Il ne s'agit toutefois pas de la seule application pour laquelle les sphéroïdes mixtes selon l'invention trouvent une utilité puisque la PCR quantitative peut être mise en oeuvre à des voies autres que la pigmentation comme l'inflammation, la prolifération, l'apoptose...

A ce titre, des essais complémentaires sur des sphéroïdes mixtes selon l'invention ont été réalisés (voir l'exemple 7) pour quantifier l'évolution de la différentiation des kératinocytes qui les composent ; cela a été réalisé à l'aide d'une puce génomique sur 64 gènes spécifiques du métabolisme des kératinocytes parmi lesquels 15 sont également exprimés par les mélanocytes, Les profils d'expression ont été comparés après 3 jours et 7 jours de culture (voir le tableau 1 de l'exemple 7). Plusieurs gènes impliqués dans l'adhésion et la cohésion épidermique sont sur-exprimés (CTNNA1, CDLN1, OCLN, EVPL, EPPK1 et PXN). Des gènes impliqués dans la différenciation et spécifiquement dans la formation de l'enveloppe cornée sont également surexprimés (AQP3, STPLC1 et TGM1). La superposition de ces deux voies cellulaires permet de confirmer les observations faites au niveau morphologique. Il est également important de noter l'augmentation d'expression de plusieurs cytokines pro-inflammatoires (IL6, IL8 et TNFA).

Alternativement ou de façon complémentaire, pour l'utilisation des sphéroïdes mixte modèles pour l'évaluation de l'activité de molécules actives, il peut s'avérer utile mais n'est pas indispensable, au cours de leur préparation, de marquer un ou plusieurs constituants de ces sphéroïdes afin de pouvoir suivre son évolution au cours du temps ou lorsque le sphéroïde sera mis en contact avec des molécules à tester. Le marquage pourra être de plusieurs natures : un marquage des cellules vivantes par un colorant avant la formation du sphéroïde ou un marquage anticorps sur coupe de sphéroïde ou sphéroïdes dissociés après formation du sphéroïde. Le type de marquage conditionnera l'analyse qui sera effectuée.

La présente invention se rapporte ainsi à l'utilisation des sphéroïdes mixtes selon l'invention pour l'évaluation d'actifs susceptibles d'agir au niveau de l'épiderme.

Comme cela a été précédemment exposé, de par la fonctionnalité de leurs mélanocytes, les sphéroïdes mixtes présentent un modèle de choix pour l'évaluation de l'activité de la pigmentation, que ce soit par inhibition ou stimulation par des molécules et/ou des mélanges de molécules formulées ou encapsulées ou des produits dermatologiques ou cosmétiques ; pour conduire cette évaluation, il est nécessaire de suivre et d'analyser la mélanogénèse. Ce suivi peut être notamment réalisé selon plusieurs axes différents : un dosage de la quantité de mélanine produite, la quantification du transfert de la mélanine produite vers les kératinocytes, le suivi de la mélanogénèse peut encore être complété d'une quantification des gènes qui lui sont liés par PCR quantitative.

En particulier, le dosage de la mélanine peut être réalisé par photométrie selon un protocole tel que celui présenté dans l'exemple 3.

L'évaluation de l'activité éventuelle d'une molécule sur la pigmentation peut également ou alternativement être réalisée par l'analyse du transfert des mélanosomes vers les kératinocytes en temps réel. Ceci peut être réalisé par un marquage tel que décrit dans l'exemple 4, puis analyse des sphéroïdes par imagerie 3D en profondeur ; cette évaluation peut également être réalisée par d'autres techniques telles que la cytométrie en flux.

L'utilisation de sphéroïdes mixtes selon l'invention comportant une masse centrale peut aussi concerner l'étude de la kératinisation et des maladies associées (par exemple, le psoriasis, la dermatite atopique...) et servir pour le criblage de molécules modulant la différenciation des kératinocytes par mesure de l'épaisseur de la masse centrale à cinétique donnée et/ou par analyse transcriptomique des gènes impliqués dans la différenciation des kératinocytes.

De façon plus générale, les sphéroïdes mixtes selon l'invention peuvent être utilisés pour l'étude de désordres ou de pathologies résultant d'un dysfonctionnement de la kératogénèse, de la mélanogénèse et/ou du transfert de mélanine ainsi que pour le criblage de molécules susceptibles de corriger ces dysfonctionnements en vue de leur utilisation pour des applications cosmétique, dermatologique ou cancérologique.

Ainsi, la présente invention vise en particulier un procédé de criblage de molécules susceptibles d'agir sur la production de mélanine et/ou sur le transfert de mélanosomes et/ou de moduler l'expression de gènes impliqués dans la mélanogénèse comprenant :
(i) la préparation de sphéroïdes éventuellement marqués par le procédé selon l'invention ; de préférence, le procédé selon l'invention comprend l'étape (d) de lavage des sphéroïdes mixtes ;
(ii) la mise en contact des sphéroïdes préparés à l'étape (i) avec une ou plusieurs molécules à tester ;
(iii) la sélection des molécules modulant la quantité de mélanine et/ou le transfert des mélanosomes et/ou l'expression de gènes impliqués dans la mélanogénèse ;
ou encore un procédé de criblage de molécules susceptibles d'agir sur la différenciation des kératinocytes comprenant :
(i) la préparation de sphéroïdes selon l'invention, par exemple comportant une masse centrale et/ou marqués ;
(ii) la mise en contact des sphéroïdes préparés à l'étape (i) avec une ou plusieurs molécules à tester ;
(iii) la sélection des molécules modulant la différenciation des kératinocytes.

### Figures

**Figure 1** **: A.** cliché illustrant un sphéroïde mixte kératinocytes-mélanocytes en lumière transmise avec un ratio cellulaire de 1 NHEM pour 4 NHEK. La barre d'échelle représente 100µm. **B.** Graphique montrant l'évolution du volume des sphéroïdes mixtes sur 10 jours.
**Figure 2** **:** Clichés représentant un sphéroïde selon l'invention à J3.
   **A.** Marquage cytokératine 5 permettant de mettre en évidence les kératinocytes.
   **B.** Marquage NKIbeteb (Pmell7) permettant de mettre en évidence les mélanocytes.
   **C.** Marquage DAPI des noyaux cellulaires.
   **D.** Marquage EdU des noyaux des cellules en prolifération. On observe des cellules proliférantes à la périphérie du sphéroïde mixte.
   **E.** Superposition des différents marquages.
   La barre d'échelle représente 100µm.
**Figure 3** : Clichés représentant un sphéroïde selon l'invention à J7.
   **A.** Marquage cytokératine 5 permettant de mettre en évidence les kératinocytes. On observe un gradient d'expression de la CK5 correspondant à un gradient de différenciation. On peut noter que la morphologie des kératinocytes se modifie en fonction du gradient de différenciation avec un allongement des cellules (flèche).
   **B.** Marquage DAPI des noyaux cellulaires. On observe une modification de la morphologie des noyaux avec un allongement (flèche) et finalement une perte des noyaux avec le gradient de différenciation.
   **C.** Marquage EdU des noyaux des cellules en prolifération. On observe des cellules proliférantes à la périphérie du sphéroïde mixte.
   **D.** Superposition des différents marquages.
   La barre d'échelle représente 100µm.
**Figure 4** : Différenciation kératinocytaire.
   **A.** Schéma d'organisation de l'épiderme.
   **B.** Coupe de peau en coloration Fontana Masson.
   **C.** Détail d'une coupe de sphéroïdes mixtes à J7 avec marquage cytokératine 5 en gris et EdU (noyaux des cellules en prolifération) en blanc.

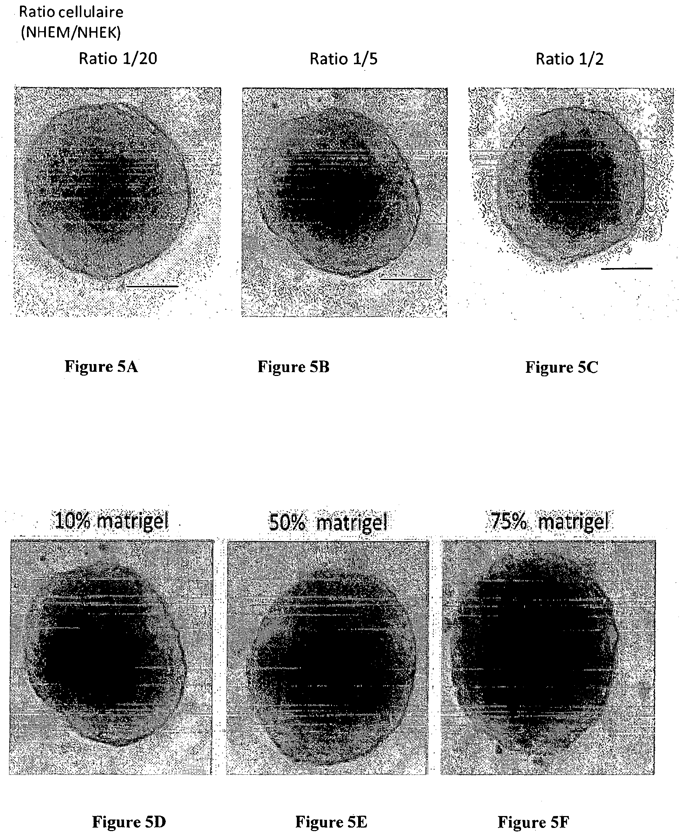

**Figure 5** : clichés de sphéroïdes selon l'invention préparés avec divers ratios mélanocyte/kératinocyte (1/20, 1/5 et 1/2). La barre d'échelle représente 100µm.

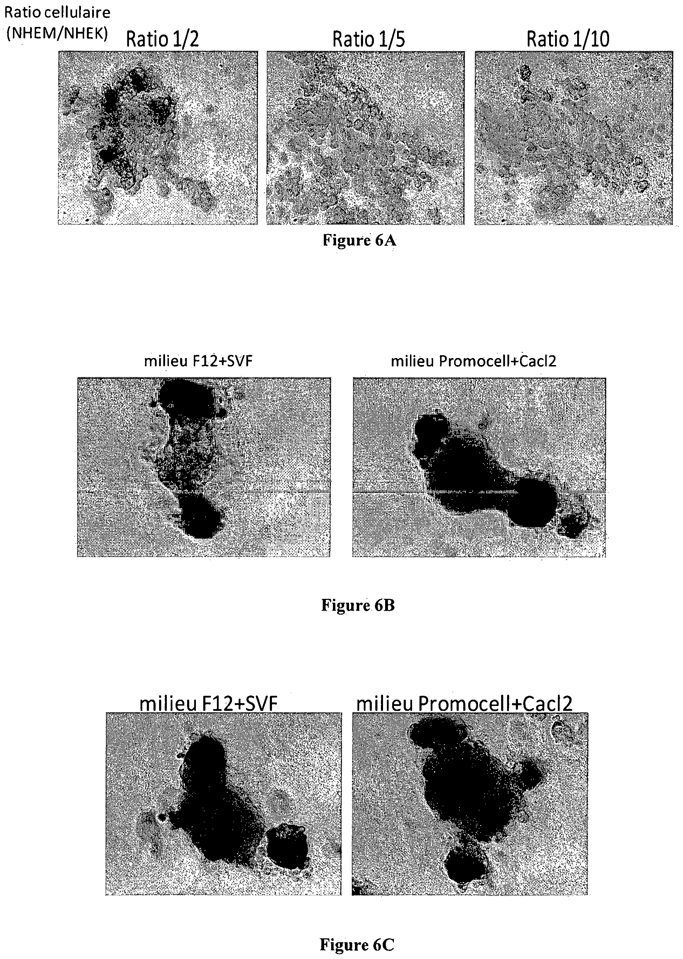

**Figures 6A** à **6E** : clichés représentant les objets obtenus par diverses tentatives de préparation de sphéroïdes telles que décrites dans l'exemple 2.

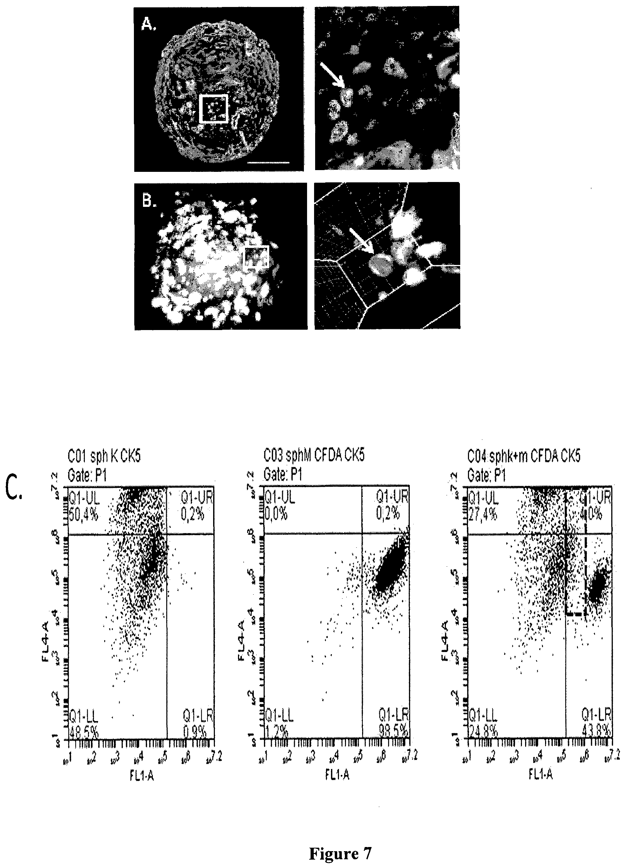

**Figure 7** : Visualisation du transfert de mélanine dans les sphéroïdes mixtes.
   **A.** Analyse d'une coupe de sphéroïdes mixtes avec marquage cytokératine 5 (gris foncé) et NKIbeteb (Pmel17) (gris clair). On observe sur le détail à droite des cellules doublement marquées (flèche). La barre d'échelle représente 100µm.
   **B.** Analyse d'un sphéroïde entier par imagerie SPIM et reconstitution 3D par le logiciel IMARIS avec marquage CellMask des kératinocytes (gris foncé) et CFDA des mélanocytes (gris clair). On observe sur le détail à droite des cellules doublement marquées (flèche).
   **C.** Analyse de sphéroïdes dissociés par cytomètrie en flux avec un marquage cytokératine 5 (FL4) et CFDA des mélanocytes (FL1). On observe une population doublement marquée (population entourée en pointillée).
**Figure 8** : histogramme représentant la concentration en mélanine sur sphéroïdes mixtes après traitement IBMX (en comparaison au contrôle solvant utilisé et PTU.
**Figure 9** : histogramme représentant l'expression génique des cellules d'un sphéroïde mixte selon l'invention quantifiée comme expliqué dans l'exemple 6. Le gène MITF directement impliqué par le traitement IBMX voit son expression augmentée. L'expression des gènes TYR, TYRP1 et PMEL sous le contrôle de MITF est également augmentée après le traitement IBMX.

### Exemple 1 - préparation de sphéroïdes selon l'invention

### Méthode

Les cellules sont décollées par trypsination et comptées afin de préparer une suspension mixte de mélanocytes et kératinocytes à 60 000 cellules/mL dans du milieu supplémenté avec 10% de Matrigel® et de 1 mM de calcium. Cette suspension est ensuite ensemencée en plaque 96 puits préalablement recouvert de polyHEMA et centrifugée 6 min à 190g à 4°C. La plaque est incubée 1h à 37°C, 5%CO₂ pour laisser figer le Matrigel® et du milieu de culture est ajouté dans chaque puits pour hydrater le Matrigel®.

### Exemple 2 - caractérisation des sphéroïdes selon l'invention

Les sphéroïdes ainsi produits ont un diamètre d'environ 250 µm après 3 jours de culture et leur volume double en 10 jours (**Figure 1**).

A J3, la méthode de marquage par le NucView (marquage de la caspase 3) montre qu'il ne se produit pas d'apoptose dans les sphéroïdes mixtes. Des marquages EdU ont permis d'établir que les cellules de la périphérie du sphéroïde sont en prolifération. Ces cellules sont également marquées à la cytokératine 5 et correspondent donc à des kératinocytes (**Figure 2**).

Il faut noter qu'après plusieurs jours en culture, une masse centrale apparaît dans le sphéroïde et que la morphologie des cellules se modifie (**Figure 3**) ; cette masse centrale est le résultat de la différenciation des kératinocytes (**Figure 4**).

Des sphéroïdes selon l'invention ont également été préparés à divers ratios cellulaires mélanocytes/kératinocytes (1/20, 1/5 et 1/2) ; tous présentent des caractéristiques satisfaisantes comme illustré aux **Figures 5A**, **5B et 5C**.

D'autres sphéroïdes selon l'invention préparés selon l'exemple 1 en utilisant 50% et 75% de Matrigel® ; là encore, les sphéroïdes mixtes obtenus présentent des caractéristiques satisfaisantes, les **Figures 5D**, **5E et 5F** sont des photographies de ces sphéroïdes préparés respectivement avec 10%, 50% et 75% de Matrigel®.

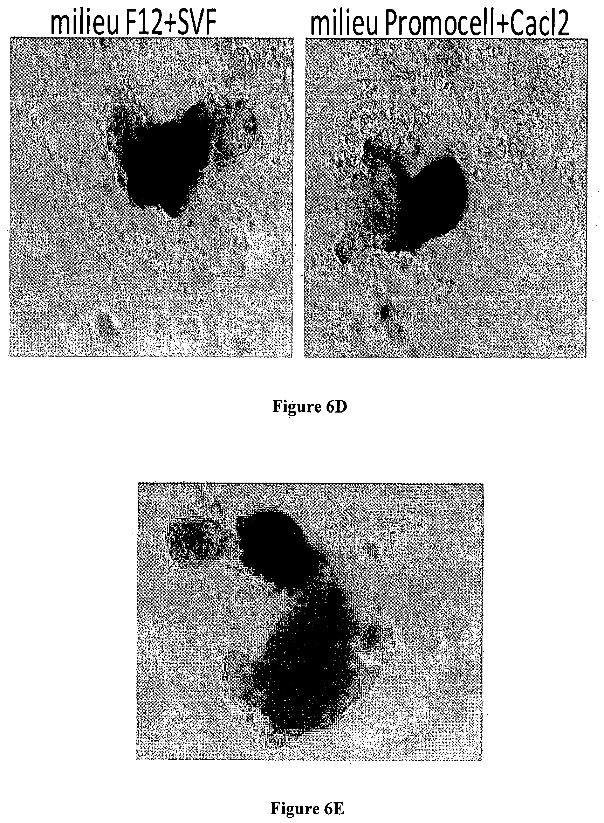

Les conditions suivantes de préparation de sphéroïdes ont également été testées, elles n'ont toutefois pas permis l'obtention de sphéroïdes :
- méthode de centrifugation d'une suspension mixte NHEM-NHEK en milieu NHEK sans Matrigel® et sans calcium ; par cette méthode, les cellules se rassemblent mais ne forment pas de structures tridimensionnelles (**Figure 6A**) ;
- méthode de centrifugation d'une suspension mixte NHEM-NHEK en milieu NHEK supplémenté avec 1mM de CaCl₂ ou en milieu F12+SVF sans Matrigel® : par cette méthode, les cellules forment des structures tridimensionnelles mais restent ségrégées par type cellulaire. La structure n'est pas sphérique et pas assez reproductible pour du criblage (**Figure 6B**) ;
- méthode de confrontation d'un sphéroïde simple de NHEK avec une suspension de NHEM en milieu NHEK supplémenté avec 1mM CaCl₂ ou en milieu F12+SVF sans Matrigel® ; les sphéroïdes simples de NHEK préalablement formés ont été réalisés par la méthode de centrifugation : par la méthode de confrontation d'un sphéroïde préexistant avec une suspension cellulaire en milieu avec calcium ou en milieu F12+SVF mais sans Matrigel®, les cellules forment des structures tridimensionnelles mais restent ségrégées par type cellulaire. La structure n'est pas sphérique et pas assez reproductible pour du criblage (**Figure 6C**) ;
- méthode de confrontation d'un sphéroïde simple de NHEM avec une suspension de NHEK en milieu NHEK supplémenté avec 1mM CaCl₂ ou en milieu F12+SVF sans Matrigel® ; les sphéroïdes simples de NHEM préalablement formés ont été réalisés par la méthode de centrifugation : par la méthode de confrontation d'un sphéroïde préexistant avec une suspension cellulaire en milieu avec calcium ou en milieu F12+SVF mais sans Matrigel®, les cellules forment des structures tridimensionnelles mais restent ségrégées par type cellulaire. La structure n'est pas sphérique et pas assez reproductible pour du criblage (**Figure 6D**) ;
- méthode de confrontation de sphéroïdes simples (sphéroïdes de NHEM d'une part et sphéroïdes de NHEK d'autre part) en milieu NHEK supplémenté avec 1mM CaCl₂ sans Matrigel® ; les deux sphéroïdes simples préalablement formés ont été réalisés par la méthode de centrifugation : par la méthode de confrontation de deux sphéroïdes de chaque type cellulaire en milieu avec calcium mais sans Matrigel®, les cellules forment des structures tridimensionnelles mais restent ségrégées par type cellulaire. La structure n'est pas sphérique et pas assez reproductible pour du criblage (**Figure 6E**) ;
- méthode de la goutte à partir d'une suspension mixte NHEM-NHEK en milieu NHEK sans calcium supplémenté ou non avec du Matrigel® (10 à 50%). Les conditions avec Matrigel® n'ont pas formées de structures 3D : par cette méthode, les cellules forment des structures tridimensionnelles mais la structure n'est pas sphérique et pas assez reproductible pour du criblage ;
- méthode de centrifugation d'une suspension mixte NHEM-NHEK en milieu NHEK sans calcium avec un pourcentage de Matrigel® variant de 10 à 50% : par cette méthode, les cellules forment des structures tridimensionnelles mais restent ségrégées par type cellulaire. La structure n'est pas sphérique et pas assez reproductible pour du criblage.

### Exemple 3 - Protocole de dosage de la mélanine par photométrie

Les cellules sont lysées dans une solution de NaOH 1M + 10% DMSO puis incubées 30min à 90°C. Une gamme standard est réalisée avec de la mélanine synthétique et la lecture est faite à une longueur d'onde de 405nm.

Le dosage des protéines totales est effectué sur le lysat du dosage de mélanine avec le kit microBCA selon les recommandations du fournisseur. Une gamme standard est réalisée avec de la BSA et la lecture est faite à une longueur d'onde de 605nm.

Les résultats sont exprimés sous la forme d'un rapport entre le dosage de mélanine et des protéines totales.

### Exemple 4 - Exemple de protocole de marquage des sphéroïdes mixtes selon l'invention

Les sphéroïdes mixtes sont préparés comme expliqué dans l'exemple 1 avec des mélanocytes préalablement marqués au CFDA (carboxy-fluorescein diacetate, succinimidyl ester). Les sphéroïdes mixtes ainsi formés sont marqués au CellMask. Ils sont ensuite lavés, fixés à la formaline, déshydratés puis transparisés dans un mélange de Benzyl-alcohol et benzyl-benzoate (BaBb). L'analyse est faite par la technique d'imagerie à feuille de lumière.

### Exemple 5 - Dosage de la mélanine sur sphéroïde mixte après traitement IBMX et PTU

24h après leur ensemencement, les sphéroïdes mixtes selon l'invention tels que préparés à l'exemple 1 ont été traités, après élimination du Matrigel® par lavage au PBS, à 300µM d'IBMX ou 500µM de PTU pendant 5 jours avec un renouvellement du traitement à 4 jours. La mélanine est ensuite dosée comme expliqué dans l'exemple 3.

Comme illustré à la **Figure 8**, les résultats obtenus montent que le traitement au PTU entraine une diminution de la quantité globale de mélanine au sein du sphéroïde mixte comparativement au contrôle solvant (EtOH) et le traitement à l'IBMX provoque une augmentation de la quantité de mélanine totale au sein du sphéroïde mixte comparativement au contrôle solvant (DMSO).

### Exemple 6 - Quantification de l'expression génique par QPCR

48h après leur ensemencement, les sphéroïdes mixtes selon l'invention tels que préparés à l'exemple 1 ont été traités, après élimination du Matrigel® par lavage au PBS, à 300µM d'IBMX pendant 24h. Les sphéroïdes ont été récupérés et lavés au PBS après 24h de traitement à l'IBMX. Les ARN ont ensuite été extraits et reverse-transcrits en cDNA. Une PCR quantitative a ensuite été réalisée pour quantifier l'expression des gènes MITF, TYR, TYRP1 et PMEL. L'expression de ces gènes a été normalisée par l'expression du gène de ménage GAPDH puis ramené au contrôle non traité.

Les résultats obtenus montrent que le gène MITF, directement impliqué par le traitement IBMX, voit son expression augmentée. L'expression des gènes TYR, TYRP1 et PMEL, sous le contrôle de MITF, est également augmentée après le traitement IBMX (**Figure 9**).

### Exemple 7 - Evaluation du profil d'expression génique par puce génomique

Le profil d'expression génique des sphéroïdes a été évalué après 3 et 7 jours de culture sur puce génomique selon le protocole détaillé ci-dessous.

Les sphéroïdes mixtes selon l'invention préparés comme décrit dans l'exemple 1 ont été récupérés, lavés au PBS respectivement à 3 jours et 7 jours après ensemencement. Les ARNm sont alors extraits puis la reverse transcription effectuée. Enfin, les étapes spécifiques de préparation pour la puce (96 gènes) selon le protocole de Fluidigm sont entreprises. Une étape de pré-amplification en présence de l'ensemble des amorces utilisées sur la puce est réalisée. Chaque échantillon d'ADNc pré-amplifié est ensuite disposé dans une plaque de 96 puits suivant le plan de plaque avec le mix permettant la PCR en temps réel. En parallèle, sur une autre plaque 96 puits, chaque paire d'amorces est disposée dans un puits suivant le plan de plaque. Ensuite chaque mix est déposé de part et d'autre de la puce. Le mélange des deux mix est effectué par l'IFC Controller puis la puce est disposée dans le BioMark pour réaliser la PCR en temps réel. La puce génomique est réalisée sur 64 gènes spécifiques du métabolisme des kératinocytes dont 15 également exprimés par les mélanocytes. Les données sont analysées grâce au logiciel Fludigm.

Les résultats obtenus sont présentés dans le tableau I ci-dessous, qui montre que plusieurs gènes impliqués dans la cohésion épidermique et la différenciation sont surexprimés suite à l'évolution du sphéroïde entre J3 et J7 comme illustré dans la **Figure 3** ; les gènes de l'inflammation sont également surexprimés :

**Tableau I : Tableau montrant les gènes sur-exprimés entre les jours 3 et 7 en culture.**

| **gène** | **taux de sur- expression** | **activité** |
|---|---|---|
| CTNNA1 | +50% | La Caténine α se lie au Cadhérine pour favoriser les interactions cellulaires. |
| CDLN1 | +280% | La claudine est une protéine membranaire intervenant dans les jonctions serrées. |
| OCLN | +270% | L'ocludine est une protéine membranaire intervenant dans les jonctions serrées. |
| EVPL | +60% | L'envoplakine est une protéine impliquée dans la formation des desmosomes. |
| EPPK1 | +540% | L'epiplakine est une protéine impliquée dans la formation des desmosomes. Elle serait également impliquée dans la différenciation cellulaire. |
| PXN | +40% | La Paxilline intervient dans des complexes de protéines appartenant au cytosquelette. Elle se trouve sur la face cytoplasmique des régions spécialisées pour l'attachement des cellules à la matrice extracellulaire. |
| AQP3 | +90% | Chez l'homme, la circulation de l'eau au sein de la peau se fait par une Aquaporine spécifique appelée AQP3. Elle joue un rôle primordial dans la formation de la barrière hydrolipidique. |
| TGM1 | +110% | La transglutaminase est impliquée dans la formation de l'enveloppe cornée en créant des cross-link entre des protéines structurales dont l'involucrine et rigidifiant ainsi la couche cornée. |
| STPLC1 | +40% | La Sérine palmitoyltransferase est une enzyme intervenant dans la biosynthèse des sphingolipides qui s'enrichissent dans la couche cornée. C'est donc un marqueur de la différenciation épidermique. |
| IL8 | +390% | Cytokine pro-inflammatoire. |
| IL6 | +710% | Cytokine pro-inflammatoire. |
| TNFA | +710% | Cytokine pro-inflammatoire. |

## Revendications

1. Procédé de préparation de sphéroïdes mixtes de kératinocytes et de mélanocytes **caractérisé en ce qu'**il comprend :
(a) la mise en suspension de kératinocytes avec des mélanocytes dans un milieu de culture supplémenté d'une préparation de protéines de la matrice extracellulaire à une concentration comprise entre 1 et 95% en volume/volume et d'une quantité inférieure ou égale à 10 mM d'un sel choisi parmi le calcium, le manganèse ou le magnésium ;
(b) la formation des sphéroïdes à partir du mélange obtenu à l'étape (a) ; et
(c) l'incubation des sphéroïdes obtenus à l'étape (b).

2. Procédé selon la revendication 1, **caractérisé en ce que** ladite suspension de l'étape (a) est préparée avec de 1 mélanocyte pour 1 kératinocyte à 1 mélanocyte pour 40 kératinocytes.

3. Procédé selon la revendication 1 ou la revendication 2, **caractérisé en ce que** les kératinocytes et les mélanocytes sont des cellules primaires saines et sont ensemencés en une quantité comprise entre 100 et 5000 cellules par sphéroïde.

4. Procédé selon l'une quelconque des revendications qui précèdent, **caractérisé en ce que** la préparation de protéines de la matrice extracellulaire est du Matrigel®.

5. Procédé selon l'une quelconque des revendications qui précèdent, **caractérisé en ce que** le sel est le calcium.

6. Procédé selon l'une quelconque des revendications qui précèdent, **caractérisé en ce que** l'incubation de l'étape (c) dure entre 1 et 10 jours.

7. Procédé selon l'une quelconque des revendications qui précèdent, **caractérisé en ce qu'**il comporte en outre une étape de marquage.

8. Procédé selon l'une quelconque des revendications qui précèdent, **caractérisé en ce qu'**il comporte une étape (d) de lavage des sphéroïdes mixtes.

9. Sphéroïde mixte de kératinocytes et de mélanocytes susceptible d'être obtenu par le procédé selon l'une quelconque des revendications 1 à 8, comprenant une préparation de protéines de la matrice extracellulaire à une concentration comprise entre 1 et 95% en volume/volume et une quantité inférieure ou égale à 10 mM d'un sel choisi parmi le calcium, le manganèse ou le magnésium.

10. Sphéroïde selon la revendication 9, **caractérisée en ce qu'**elle a un diamètre apparent compris entre 100 et 1000 µm et une forme régulière.

11. Sphéroïde selon la revendication 9 ou la revendication 10, **caractérisée en ce qu'**elle comporte une masse centrale.

12. Sphéroïde selon l'une quelconque des revendications 9 à 11, **caractérisé en ce qu'**au moins un de ses constituants est marqué.

13. Utilisation d'un sphéroïde selon l'une quelconque des revendications 9 à 12 pour évaluer l'activité de composés agissant au niveau de l'épiderme.

14. Utilisation d'un sphéroïde selon l'une quelconque des revendications 9 à 12 pour tester l'activité de composés sur la production de mélanine et/ou le transfert des mélanosomes et/ou l'expression de gènes impliqués dans la mélanogénèse.

15. Utilisation d'un sphéroïde selon l'une quelconque des revendications 9 à 12 pour tester l'activité de composés sur la différenciation des kératinocytes.

16. Procédé de criblage de molécules susceptibles d'agir sur la production de mélanine et/ou sur le transfert de mélanosomes et/ou sur l'expression de gènes impliqués dans la mélanogénèse comprenant :
(i) la préparation de sphéroïdes selon l'une des revendications 9 à 12 ;
(ii) la mise en contact des sphéroïdes préparés à l'étape (i) avec une ou plusieurs molécules à tester ;
(iii) la sélection des molécules modulant la production de mélanine et/ou le transfert de mélanosomes et/ou l'expression de gènes impliqués dans la mélanogénèse.

17. Procédé de criblage de molécules susceptibles d'agir sur la différenciation des kératinocytes comprenant :
(i) la préparation de sphéroïdes selon l'une des revendications 9 à 12 ;
(ii) la mise en contact des sphéroïdes préparés à l'étape (i) avec une ou plusieurs molécules à tester ;
(iii) la sélection des molécules modulant la différenciation des kératinocytes.

## Patentansprüche

1. Verfahren zur Zubereitung von gemischten Sphäroiden aus Keratinozyten und aus Melanozyten, **dadurch gekennzeichnet, dass** es umfasst:
(a) das Suspendieren von Keratinozyten mit Melanozyten in einem Kulturmedium, das mit einer Zubereitung aus Proteinen der extrazellulären Matrix in einer Konzentration im Bereich zwischen 1 und 95 % Volumen/Volumen und einer Menge kleiner oder gleich 10 mM eines Salzes angereichert ist, ausgewählt aus Calcium, Mangan oder Magnesium;
(b) das Bilden der Sphäroide ausgehend von dem im Schritt (a) erhaltenen Gemisch; und
(c) das Inkubieren der im Schritt (b) erhaltenen Sphäroide.

2. Verfahren nach Anspruch 1, **dadurch gekennzeichnet, dass** die Suspension aus dem Schritt (a) mit von 1 Melanozyten auf 1 Keratinozyten bis 1 Melanozyten auf 40 Keratinozyten zubereitet wird.

3. Verfahren nach Anspruch 1 oder Anspruch 2, **dadurch gekennzeichnet, dass** die Keratinozyten und die Melanozyten gesunde Primärzellen sind und in einer Menge im Bereich zwischen 100 und 5000 Zellen pro Sphäroid geimpft werden.

4. Verfahren nach einem der vorstehenden Ansprüche, **dadurch gekennzeichnet, dass** die Zubereitung aus Proteinen der extrazellulären Matrix Matrigel® ist.

5. Verfahren nach einem der vorstehenden Ansprüche, **dadurch gekennzeichnet, dass** das Salz Calcium ist.

6. Verfahren nach einem der vorstehenden Ansprüche, **dadurch gekennzeichnet, dass** das Inkubieren aus dem Schritt (c) zwischen 1 und 10 Tagen dauert.

7. Verfahren nach einem der vorstehenden Ansprüche, **dadurch gekennzeichnet, dass** es weiter einen Schritt des Markierens aufweist.

8. Verfahren nach einem der vorstehenden Ansprüche, **dadurch gekennzeichnet, dass** es einen Schritt (d) des Waschens der gemischten Sphäroide aufweist.

9. Gemischtes Sphäroid aus Keratinozyten und aus Melanozyten, das über das Verfahren nach einem der Ansprüche 1 bis 8 erhalten werden kann, umfassend eine Zubereitung aus Proteinen der extrazellulären Matrix in einer Konzentration im Bereich zwischen 1 und 95 % Volumen/Volumen und einer Menge kleiner oder gleich 10 mM eines Salzes, ausgewählt aus Calcium, Mangan oder Magnesium.

10. Sphäroid nach Anspruch 9, **dadurch gekennzeichnet, dass** es einen scheinbaren Durchmesser im Bereich zwischen 100 und 1000 µm und eine regelmäßige Form hat.

11. Sphäroid nach Anspruch 9 oder Anspruch 10, **dadurch gekennzeichnet, dass** es eine zentrale Masse aufweist.

12. Sphäroid nach einem der Ansprüche 9 bis 11, **dadurch gekennzeichnet, dass** mindestens einer seiner Bestandteile markiert ist.

13. Verwendung eines Sphäroids nach einem der Ansprüche 9 bis 12, um die Wirkung von Verbindungen zu bewerten, die im Bereich der Epidermis wirken.

14. Verwendung eines Sphäroids nach einem der Ansprüche 9 bis 12, um die Wirkung von Verbindungen auf die Produktion von Melanin und/oder den Transfer der Melanosomen und/oder die Expression von Genen zu testen, die an der Melanogenese beteiligt sind.

15. Verwendung eines Sphäroids nach einem der Ansprüche 9 bis 12, um die Wirkung von Verbindungen auf die Differenzierung der Keratinozyten zu testen.

16. Verfahren zum Screenen von Molekülen, die auf die Produktion von Melanin und/oder auf den Transfer von Melanosomen und/oder auf die Expression von Genen wirken können, welche an der Melanogenese beteiligt sind, umfassend:
(i) das Zubereiten von Sphäroiden nach einem der Ansprüche 9 bis 12;
(ii) das Inkontaktbringen der im Schritt (i) zubereiteten Sphäroide mit einem oder mehreren Molekülen, die getestet werden sollen;
(iii) das Auswählen der Moleküle, die die Produktion von Melanin und/oder den Transfer von Melanosomen und/oder die Expression von Genen modulieren, die an der Melanogenese beteiligt sind.

17. Verfahren zum Screenen von Molekülen, die auf die Differenzierung der Keratinozyten wirken können, umfassend:
(i) das Zubereiten von Sphäroiden nach einem der Ansprüche 9 bis 12;
(ii) das Inkontaktbringen der im Schritt (i) zubereiteten Sphäroide mit einem oder mehreren Molekülen, die getestet werden sollen;
(iii) das Auswählen der Moleküle, die die Differenzierung der Keratinozyten modulieren.

## Claims

1. Method for preparing mixed spheroids of keratinocytes and melanocytes, **characterised in that** it comprises:
(a) the placing in a suspension of keratinocytes with melanocytes in a culture medium supplemented with a preparation of proteins of the extracellular matrix at a concentration of between 1 and 95% by volume/volume and a quantity less than or equal to 10mM of a salt selected from calcium, manganese or magnesium;
(b) the formation of spheroids from the mixture obtained in step (a); and
(c) the incubation of the spheroids obtained in step (b).

2. Method according to claim 1, **characterised in that** said suspension of step (a) is prepared with 1 melanocyte for 1 keratinocyte at to 1 melanocyte for 40 keratinocytes.

3. Method according to claim 1 or to claim 2, **characterised in that** the keratinocytes and melanocytes are healthy primary cells and are seeded in a quantity of between 100 and 5000 cells per spheroid.

4. Method according to any one of the preceding claims, **characterised in that** the preparation of proteins of the extracellular matrix is Matrigel®.

5. Method according to any one of the preceding claims, **characterised in that** the salt is calcium.

6. Method according to any one of the preceding claims, **characterised in that** the incubation of step (c) lasts between 1 and 10 days.

7. Method according to any one of the preceding claims, **characterised in that** it further comprises a marking step.

8. Method according to any one of the preceding claims, **characterised in that** it comprises a step (d) of washing the mixed spheroids.

9. Mixed spheroid of keratinocytes and melanocytes likely to be obtained by the method according to any one of claims 1 to 8, comprising a preparation of proteins of the extracellular matrix at a concentration of between 1 to 95% by volume/volume and a quantity less than or equal to 10mM of a salt selected from calcium, manganese or magnesium.

10. Spheroid according to claim 9, **characterised in that** it has a visible diameter of between 100 and 1000 µm and a regular shape.

11. Spheroid according to claim 9 or to claim 10, **characterised in that** it comprises a central mass.

12. Spheroid according to any one of claims 9 to 11, **characterised in that** at least one of the components thereof is marked.

13. Use of a spheroid according to any one of claims 9 to 12 to evaluate the activity of compounds acting at the level of the epidermis.

14. Use of a spheroid according to any one of claims 9 to 12 to test the activity of compounds on the production of melanin and/or the transfer of melanosomes and/or the expression of genes involved in the melanogenesis.

15. Use of a spheroid according to any one of claims 9 to 12 to test the activity of compounds on the differentiation of the keratinocytes.

16. Method for screening molecules likely to act on the production of melanin and/or on the transfer of melanosomes and/or on the expression of genes involved in the melanogenesis comprising:
(i) the preparation of spheroids according to one of claims 9 to 12;
(ii) the contacting of the spheroids prepared in step (i) with one or more molecules to be tested;
(iii) the selection of molecules modulating the production of melanin and/or the transfer of melanosomes and/or the expression of genes involved in the melanogenesis.

17. Method for screening molecules likely to act on the differentiation of the keratinocytes comprising:
(i) the preparation of spheroids according to one of claims 9 to 12;
(ii) the contacting of the spheroids prepared in step (i) with one or more molecules to be tested;
(iii) the selection of molecules modulating the differentiation of the keratinocytes.
